# EUROPÄISCHE PATENTANMELDUNG

(11) **EP 2 347 778 A1**
(43) Veröffentlichungstag der Anmeldung: **27.07.2011**
(21) Anmeldenummer: 10075040.5
(22) Anmeldetag: 25.01.2010
(51) Int. Cl.: A61M 1/10, F04D 29/18, F04D 29/24, F04D 3/02

(54) **Fluidpumpe mit einem radial komprimierbaren Rotor**

(71) Anmelder: ECP Entwicklungsgesellschaft mbH, 12247 Berlin (DE)
(72) Erfinder: Töllner, Thomas, 12047 Berlin (DE)
(74) Vertreter: Pfenning, Meinig & Partner GbR

(57) **Zusammenfassung**

Um bei einer Fluidpumpe, insbesondere für den mikroinvasiven medizinischen Einsatz den Rotor (6, 6', 6'', 6''', 60, 60') in radialer Richtung kompressibel zu gestalten, wird dieser in seiner Längsrichtung (16) durch axial auf ihn wirkende Schub- und Zugelemente dehnbar ausgeführt.

## Beschreibung

Die Erfindung liegt auf dem Gebiet des Maschinenbaus, insbesondere der Mikromechanik, und bezieht sich speziell auf Fluidpumpen mit einem Rotor und wenigstens einem Schaufelblatt zur überwiegend axialen Förderung eines Fluids.

Derartige Pumpen können in verschiedenen technischen Gebieten eingesetzt werden, besonders dort, wo Einsatzorte schwierig zugänglich sind und ein komprimierbarer Rotor in komprimierter Form an Ort und Stelle gebracht werden und dort für einen effizienten Betrieb expandiert werden kann.

Eine besonders vorteilhafte Anwendung liegt auf dem Gebiet der Medizin, wo derartige Pumpen in besonders kleiner Bauform beispielsweise durch Blutgefäße in den Körper eines Patienten eingeführt, am Einsatzort, vorzugsweise in einer Herzkammer, expandiert und dort betrieben werden können. Zum Entfernen der Pumpe kann diese üblicherweise wieder komprimiert und durch eine Schleuse ausgebracht werden.

Entsprechende komprimierbare Pumpen sind in verschiedenen Bauformen bereits bekannt.

Beispielsweise ist aus der WO 03/103745 A2 ein Rotor bekannt, der in einem komprimierten Zustand einen geringeren Durchmesser aufweist als in einem expandierten Zustand und der ein entfaltbares Rotorblatt aufweist, das sich im Betrieb durch den Fluidgegendruck des Fluids entfaltet.
Die WO 03/103745 offenbart zudem einen Rotor, der axial gegeneinander verschiebbare Elemente aufweist, deren gegenseitige Verschiebung mit der Expansion und Kompression eines den Rotor gehäuseartig umgebenden Käfigs einhergeht.

Aus dem Stand der Technik sind zudem Rotoren bekannt, deren Schaufelblätter zum Betrieb entfaltbar sind und zu diesem Zweck Gelenke oder elastische Stützteile aufweisen. Insbesondere ist die Verwendung von sogenannten Gedächtnislegierungen wie beispielsweise Nitinol bekannt, die abhängig von der Umgebungstemperatur verschiedene geometrische Formen annehmen und dadurch eine nachträgliche Verformung des Rotors nach dem Einbringen in einen Körper ermöglichen.

Aus der WO 99/44651 ist eine Fluidpumpe mit einem komprimierbaren Rotor bekannt, der eine komprimierbare, mit einer Membran bespannte Wendel aus einer Gedächtnislegierung aufweist, die durch ein elastisches Band axial zusammengehalten ist. Durch radialen Druck auf die Wendel kann diese komprimiert werden.

Aus der WO 94/05347 ist eine Fluidpumpe bekannt mit einem Förderelemente tragenden Rotor, der fest mit einer Welle verbunden ist wobei auf der Welle zudem eine Hülse axial verschiebbar angeordnet ist, durch deren Verschiebung ein den Rotor umgebendes Gehäuse in die Länge gezogen und damit radial komprimiert werden kann. Durch eine Hebelmechanik wird eine Relativbewegung der Hülse zur Welle in eine radiale Aufrichtung der Förderelemente oder ein Anklappen an die Welle umgesetzt.

Die Erfahrung zeigt, dass komplexe Konstruktionen für derartige Pumpen auch wegen der kleinen Bauform schwierig zu realisieren und mit den gewünschten Standzeiten zuverlässig zu betreiben sind.

Der vorliegenden Erfindung liegt vor dem Hintergrund des Standes der Technik daher die Aufgabe zugrunde, eine Fluidpumpe der eingangs genannten Art zu schaffen, die mit einfachem konstruktivem Aufbau und bei geringen Kosten eine zuverlässige Funktion der Pumpe sowohl beim Einbringen und Ausbringen als auch im regulären Betrieb erlaubt.

Die Aufgabe wird mit den Merkmalen der Erfindung gemäß Patentanspruch 1 gelöst.

Dabei sieht die erfindungsgemäße Fluidpumpe einen um seine Längsachse drehbaren, antreibbaren Rotor mit wenigstens einer Förderschaufel vor, wobei der Rotor wenigstens teilweise aus einem elastisch komprimierbaren und expandierbaren Material besteht und durch axial auf ihn wirkende Elemente in Richtung seiner Längsachse elastisch dehnbar ist.

Es kann dabei zudem vorgesehen sein, dass ein Schub-und ein Zugelement an verschiedenen Enden des Rotors und/oder eines Gehäuses der Fluidpumpe angreifen.

Durch die elastische Dehnbarkeit in Längsrichtung kann bei einem derartigen materialelastischen Rotor, der teilweise zum Besipiel aus einem Elastomer oder einem Schaumstoff bestehen kann, einerseits direkt eine Querkontraktion realisiert werden. Es können aber auch durch die Längsdehnung verschiedene Mechanismen innerhalb des Rotors in Gang gesetzt werden, die zu einer Querkompression oder einer leichteren Querkomprimierbarkeit führen. Hierzu kann z. B. vorgesehen sein, dass die Schaufelblätter in Längsrichtung des Rotors mit gedehnt werden, dadurch dass sie über eine gewisse Strecke in Längsrichtung der Nabe mit dieser verbunden sind. Die Schaufelblätter können im ungedehnten Zustand eine konkave oder konvexe oder gefaltete Form oder entsprechend geformte Stützstrukturen in ihrem Inneren aufweisen, die durch die Längsdehnung des Rotors gestreckt werden. Derartige geometrische Formen können im ungedehnten Zustand den Schaufeln zusätzliche Stabilität gegenüber dem Fluiddruck im Betrieb, jedoch auch gegenüber einer radialen Kompression verleihen. Werden derartige Strukturen durch Längsdehnung des Rotors gestreckt, so ergibt sich eine erleichterte Verformbarkeit der Schaufelblätter, so dass der Rotor insgesamt entweder leichter radial komprimierbar wird oder bei einer Längsdehnung sich selbsttätig wenigstens ein Stück weit komprimiert.

Eine entsprechende Erleichterung der radialen Komprimierbarkeit kann dann gegebenenfalls durch zusätzliche Mechanismen der Radialkompression, beispielsweise beim Einfahren in eine Schleuse, genutzt werden.

Die beschriebene Konstruktion mit einer in Längsrichtung ausgedehnten Verbindung zwischen Schaufelblättern und Nabe ist zudem insofern einfach aufgebaut, als Rotor und Schaufelblätter einstückig hergestellt, beispielsweise gegossen oder vulkanisiert werden können.

Für den medizinischen Einsatz einer erfindungsgemäßen Fluidpumpe kann vorteilhaft der Rotor am distalen Ende eines Hohlkatheters angeordnet sein, um ihn mit diesem durch eine Blutbahn beispielsweise in eine Herzkammer transportieren zu können.

In diesem Fall kann vorteilhaft vorgesehen sein, dass durch zwei entlang des Katheters verlaufende, gegeneinander bewegbare Elemente auf beide Enden des Rotors ein Zug in einander entgegengesetzten Richtungen aufbringbar ist. Entlang des Katheters kann somit durch verschiedene gegeneinander verschiebbare Elemente der Rotor vom proximalen Ende des Katheters aus gedehnt werden.

Dabei kann vorteilhaft vorgesehen sein, dass eines der Elemente eine Hülle und das andere Element eine in der Hülle verlaufende Seele ist. Beim Vorsehen einer Hülle und einer darin verlaufenden Seele können diese beiden Elemente sich gegenseitig gegen Ausknicken stützen. Es kann einerseits mittels der Hülle ein Zug aufgebracht werden und mittels der Seele ein Druck in Längsrichtung des Katheters oder umgekehrt. Vorteilhaft kann der Hohlkatheter selbst als Hülle dienen und gegebenenfalls auch eine in diesem verlaufende Antriebswelle als Seele.

Es können jedoch alternativ oder zusätzlich dazu auch Züge beispielsweise am äußeren Umfang des Katheters eng geführt sein, die bei entsprechender Steifigkeit sowohl einen Zug als auch einen Druck in Längsrichtung bzw. entsprechende Zug- und Schubbewegungen übertragen können.

Diese Züge können entlang des Katheters bis zu der Schleuse, durch die der Katheter in den Körper eingeführt ist, und weiter zum Körperäußeren verlaufen und dort in einem Betätigungsring befestigt sein, so dass die Züge von außerhalb des Patientenkörpers komfortabel bedienbar sind.

Zur konkreteren Ausgestaltung der Erfindung kann vorgesehen sein, dass das proximale Ende des Rotors mit der Seele und das distale Ende des Rotors mit einem Gehäuse des Rotors zugfest verbunden sind und dass das Gehäuse mit der Hülle druckfest verbunden ist. In diesem Fall kann die Seele auf den Rotor an dessen proximalem Ende einen Zug aufbringen, während das andere, distale Ende des Rotors in einem Gehäuse, beispielsweise in einem zugfesten Drehlager, gehalten ist, das einen Zug in einer der Seele entgegengesetzten Richtung auf den Rotor aufbringt, der durch eine Stützung des Gehäuses am distalen Ende des Hohlkatheters/der Hülle als Haltekraft realisierbar ist. Es ist dann auf die Hülle eine Druckkraft entlang des Katheters aufzubringen und auf die Seele eine entsprechende Zugkraft.

Es kann vorteilhaft auch vorgesehen sein, dass das proximale Ende des Rotors mit dem Ende der Hülle unmittelbar oder mittelbar über das Gehäuse zugfest verbunden ist und dass das distale Ende des Rotors mit der Seele unmittelbar oder mittelbar über das Gehäuse druckfest verbunden ist. In diesem Fall wird auf das proximale Ende des Rotors durch das distale Ende der Hülle eine Zugkraft aufgebracht, beispielsweise durch eine zugfeste Drehlagerung, während auf das distale Ende des Rotors durch die Seele, die eine entsprechende Schubkraft überträgt, eine Zugkraft in distaler Richtung aufgebracht wird. Die Seele kann zu diesem Zweck beispielsweise durch den Rotor hindurch verlaufen und an dessen distalem Ende mit dem Rotor verbunden sein. Die Seele kann beispielsweise durch eine Antriebswelle des Rotors gebildet sein.

Eine weitere Ausführungsform der Erfindung sieht vor, dass der Rotor von einem ebenfalls in Richtung der Längsachse dehnbaren Gehäuse umgeben ist. In diesem Fall kann der Rotor an seinen beiden Enden jeweils mit dem Gehäuse drehbar, jedoch zugfest verbunden sein, z. B. dadurch, dass der Rotor beidseitig im Gehäuse in zugfesten Drehlagern gelagert ist. Wird dann auf das Gehäuse eine Längsdehnung ausgeübt, so überträgt sich diese direkt auf den Rotor. Damit wird der Rotor entweder direkt in Radialrichtung komprimiert, oder er wird jedenfalls leichter komprimierbar.

Besonders vorteilhaft kann dabei vorgesehen sein, dass das Gehäuse im Falle einer Querkompression im Wesentlichen senkrecht zur Längsachse selbsttätig eine Längsdehnung parallel zur Längsachse erfährt. Es kann dabei vorteilhaft vorgesehen sein, dass das Gehäuse in dem Zustand, in dem keine Kräfte auf es in Längsrichtung wirken, eine bauchige Form mit einem für den expandierten Rotor ausreichenden Innenraum aufweist.

In diesem Fall kann durch Aufbringen einer Radialkompression auf das Gehäuse, also beispielsweise durch Einfahren des Gehäuses in eine trichterförmige Schleuse, eine Längsdehnung des Gehäuses und damit eine Längsdehnung des Rotors, verbunden mit einer Querkontraktion bzw. Erleichterung der Kompressibilität des Rotors, erreicht werden. Zu diesem Zweck kann beispielsweise am Ende des Hohlkatheters eine entsprechende Schleuse vorgesehen sein, die beim Zurückziehen des Pumpengehäuses eine entsprechende Radialkompression erzeugt. Es kann jedoch auch eine Schleuse vorgesehen sein, die den Hohlkatheter als solchen umgibt und in die der Hohlkatheter zur Kompression des Gehäuses zurückziehbar ist. Eine entsprechende Schleuse kann zu diesem Zweck einen Einlauftrichter für das Pumpengehäuse aufweisen.

Die Erfindung wird im Folgenden anhand eines Ausführungsbeispiels in einer Zeichnung gezeigt und anschließend beschrieben.

Dabei zeigt
- Fig. 1: in einer Übersicht schematisch den Einsatz einer erfindungsgemäßen Mikropumpe in einer Herzkammer,
- Fig. 2: eine dreidimensionale Ansicht eines erfindungsgemäßen Rotors in ungedehnter Form,
- Fig. 3: die Ansicht des Rotors aus Fig. 2 in längsgedehnter Form,
- Fig. 4: eine Ansicht eines weiteren Rotors in ungedehnter Form,
- Fig. 5: den Rotor aus Fig. 4 in einer in Längsrichtung gedehnten Form,
- Fig. 6: einen weiteren Rotor in einer dreidimensionalen Ansicht in ungedehnter Form,
- Fig. 7: den Rotor aus Fig. 6 in einer in Längsrichtung gedehnten Form,
- Fig. 8: im Längsschnitt stark vergrößert den Aufbau einer erfindungsgemäßen Mikropumpe mit dem Ende eines Hohlkatheters,
- Fig. 9: eine Befestigungsvorrichtung für Züge, die entlang des Katheters zur Manipulation der Pumpe laufen,
- Fig. 10: die Anordnung aus Fig. 9 in verkeiltem Zustand,
- Fig. 11: einen Längsschnitt durch einen Rotor, in dessen zentrischem Hohlraum eine Antriebswelle verläuft,
- Fig. 12: schematisch in einer ersten Anordnung die Bauteile einer Pumpe, die am Aufbringen eines Längszuges auf den Rotor beteiligt sind,
- Fig. 13: eine ähnliche Anordnung wie in Fig. 12 mit einem anderen Prinzip zur Aufbringung des Zuges sowie
- Fig. 14: eine dritte Anordnung zur Aufbringung eines Längszuges auf einen Rotor unter Anwendung eines weiteren Prinzips.

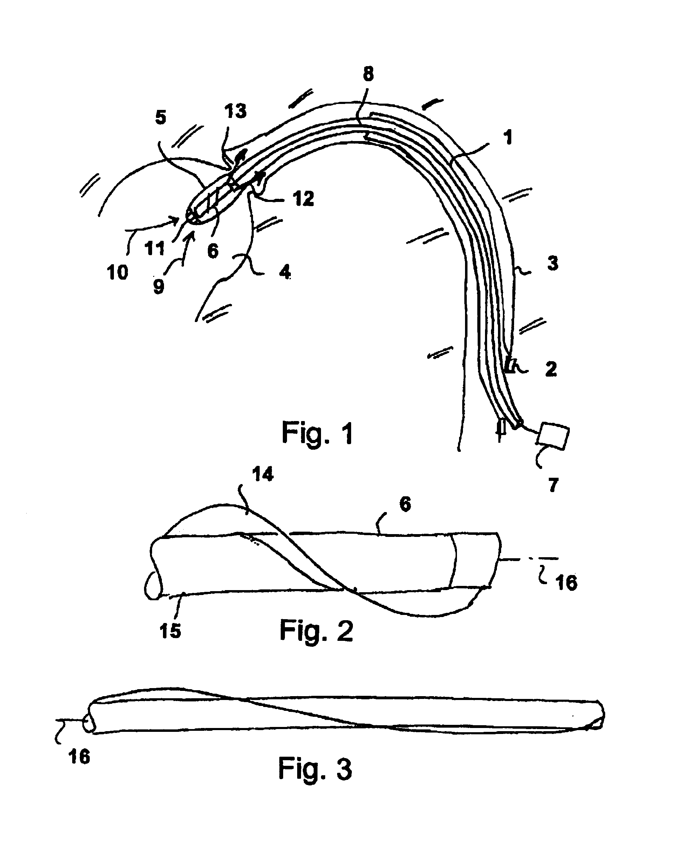

Fig. 1 zeigt einen Hohlkatheter 1, der durch eine Schleuse 2 in ein Blutgefäß 3 eines menschlichen Körpers eingeführt und dort bis zu der Herzkammer 4 eingeschoben ist. Am distalen Ende des Hohlkatheters 1 ist eine Pumpe 5 mit einem Rotor 6 befestigt, der um seine Längsachse rotiert und damit Blut aus der Herzkammer 4 axial in das Blutgefäß 3 fördert.

Der Rotor ist dazu von einem Motor 7 über eine Welle 8 mit hoher Drehzahl, typisch zwischen 10000 und 50000 Umdrehungen pro Minute antreibbar.

Durch die Rotation des Rotors 6 wird Blut axial in Richtung der Pfeile 9, 10 durch die Ansaugöffnungen 11 der Pumpe angesaugt und in Richtung der Pfeile 12, 13 innerhalb des Blutgefäßes 3 wieder ausgestoßen. Dadurch wird die Herztätigkeit bei der Blutförderung ersetzt oder ergänzt.

Die Pumpe 5 weist ein den Rotor 6 umgebendes Gehäuse auf und ist im Ganzen in Bezug auf den Durchmesser radial komprimierbar zum Einschieben in das Blutgefäß 3.

Nachdem die Pumpe 5 die Herzkammer 4 erreicht hat, kann sie radial expandiert werden, indem sowohl das Gehäuse als auch der Rotor 6 expandiert wird, um eine höhere Leistungsfähigkeit der Pumpe durch Aufstellen der Rotorblätter zu erreichen.

Die vorliegende Erfindung hat sich zur Aufgabe gemacht, eine möglichst gute und einfache radiale Komprimierbarkeit des Rotors 6 zu erreichen.

Um die Funktion der Erfindung zu illustrieren sei zunächst anhand der Fig. 2 ein Rotor 6 mit einem wendelförmig umlaufenden Schaufelblatt 14 betrachtet. Das Schaufelblatt 14 muss eine bestimmte Mindeststeifigkeit aufweisen, um im Betrieb nicht beim Fördern eines Fluids durch den Fluidgegendruck an die Nabe 15 des Rotors 6 angeklappt zu werden.

Diese Steifigkeit macht es grundsätzlich schwierig, eine radiale Kompression bzw. ein Anlegen des Schaufelblatts 14 an die Nabe 15 zur Durchmesserreduktion des Rotors bei Installation der Pumpe zu erreichen.

In der Fig. 3 ist der Rotor aus Fig. 2 in einer in Längsrichtung gedehnten Form dargestellt. Der Nabenkörper 15 kann, ebenso wie die Schaufelblätter, beispielsweise aus einem Gummi oder einem anderen Elastomer oder einem Schaumstoff oder einem anderen kompressiblen und expandierbaren Werkstoff bestehen und komprimiert sich durch den grundsätzlichen Volumenerhalt selbsttätig bei einer Längsdehnung des Rotors.

Gleichzeitig verringern sich die Ausmaße des Schaufelblatts 14 quer zur Längsrichtung 16, so dass durch gleichzeitige radiale Kompression des Nabenkörpers 15 und des Schaufelblatts 14 die Gesamtmaße des Rotors 6 quer zur Längsrichtung 16 abnehmen. Der Rotor ist in diesem Zustand wesentlich leichter durch ein schmales Blutgefäß transportierbar als im expandierten Zustand ohne eine Längsdehnung des Rotors. Damit reduziert sich ohne weiteres der Gesamtdurchmesser des Rotors. Zusätzlich kann sich die Längsdehnung noch auf die Schaufelblätter auswirken.

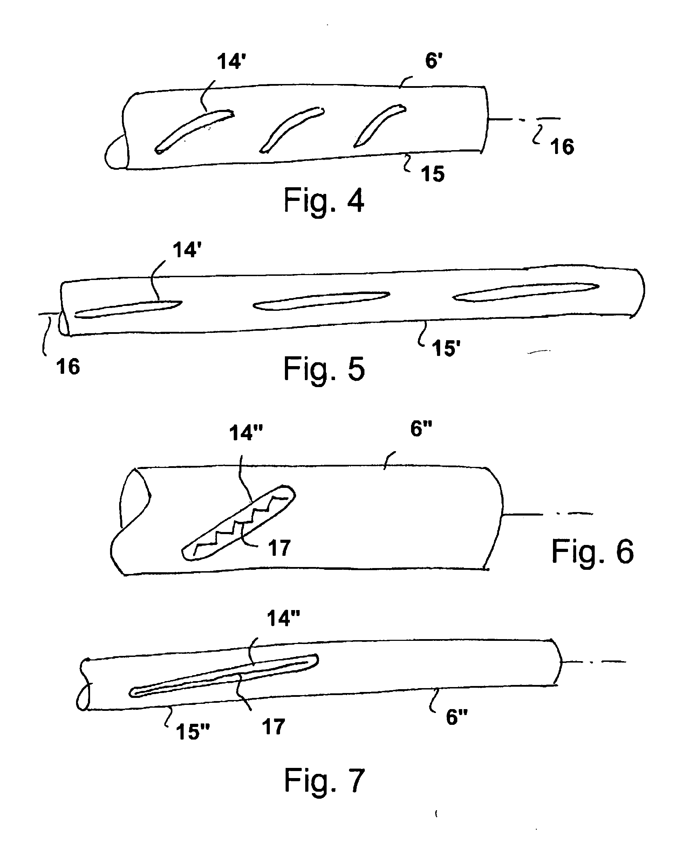

Fig. 4 zeigt eine andere Ausgestaltung des Rotors 6' mit Schaufelblättern 14', die im Querschnitt konkav oder gebogen ausgebildet sind, um dem einzelnen Schaufelblatt zusätzliche Stabilität gegenüber einem Einknicken durch den Fluidgegendruck zu verleihen.

Wird der entsprechende Rotor 6' in Längsrichtung 16 gedehnt, so zeigt sich das Bild wie in Fig. 5 dargestellt, bei dem der Durchmesser des Nabenkörpers 15' reduziert ist und gleichzeitig die Schaufelblätter 14' in Längsrichtung 16 langgezogen sind. Hierdurch wird vollständig oder fast vollständig die konkave Form der Schaufelblätter 14' beseitigt, so dass die Stabilität jedes einzelnen Schaufelblattes gegenüber einer Knickbewegung in Umfangsrichtung des Rotors deutlich herabgesetzt ist. Die Stabilität der Schaufelblätter ist somit gemindert und eine radiale Kompression durch äußere Einwirkung, beispielsweise bei einer Kompression des den Rotor umgebenden Gehäuses ist vereinfacht.

In Fig. 6 ist ein anderes Prinzip des Rotoraufbaus dargestellt, das zusätzlich oder alternativ zu den oben geschilderten Aufbauten verwendet werden kann, wobei ein Rotor 6" mit Schaufelblättern 14" ausgestattet ist, die in ihrem Inneren eine Versteifungsstruktur 16 in Form eines im Querschnitt sägezahnförmig geknickten Bleches oder anderen Flachmaterials aufweisen. Dieses geknickte Verstärkungsmaterial versteift das Schaufelblatt 14" stark gegenüber einer Verknickung.

Wird der Rotor 6" in die Länge gezogen, so ergibt sich die Situation wie sie in Fig. 7 dargestellt ist, wobei das Schaufelblatt 14" in die Länge gezogen ist und damit der Anstellwinkel verkleinert ist und wobei gleichzeitig die Verstärkungsstruktur 17 durch die Dehnung in Längsrichtung bis zur vollständigen Eliminierung der Knicke langgezogen ist.

Hierdurch wird das Schaufelblatt 14" wesentlich leichter an den Nabenkörper 15" anklappbar und der Rotor 6" wird damit einerseits bezüglich des Nabenkörpers radial komprimiert und bezüglich der Schaufelblätter einfacher noch weiter komprimierbar.

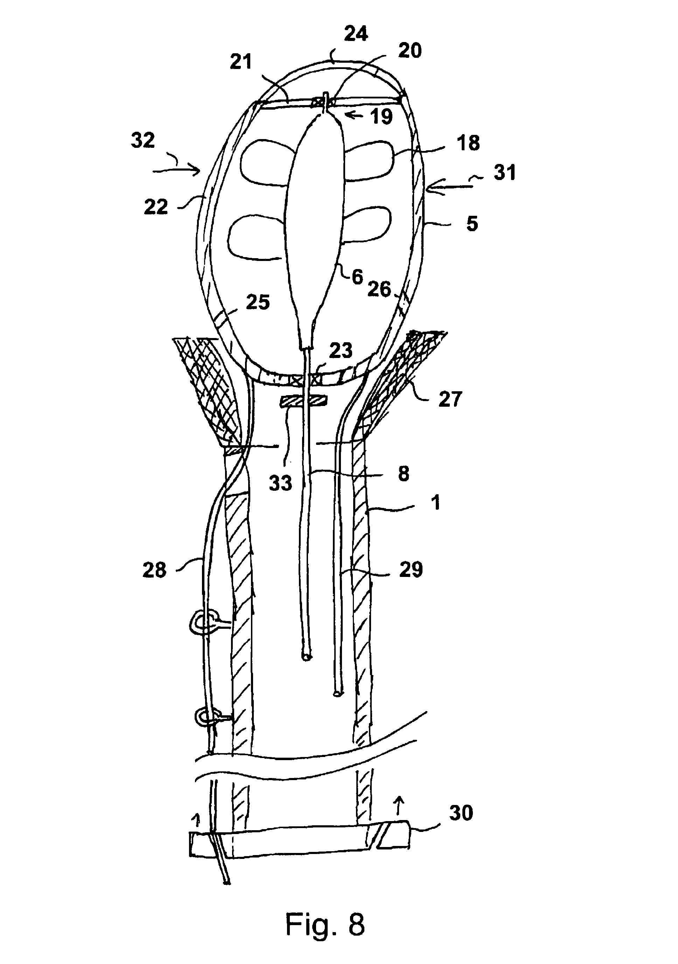

Fig. 8 zeigt in einem Längsschnitt eine Fluidpumpe 5 mit einem Rotor 6, der Schaufelblätter 18 aufweist. Schematisch ist dargestellt, dass der Rotor 6 an seinem distalen Ende 19 in einem distalen Lager 20 drehbar gelagert ist. Das Lager 20 ist an Streben 21 des Pumpengehäuses 22 befestigt.

Der Rotor 6 ist an seinem proximalen Ende in einem proximalen Lager 23 drehbar gelagert, und zwar mittels einer Welle 8 bzw. eines versteiften Anschlussstückes der Welle 8 an dem Rotor 6.

Die Fluidpumpe 5 saugt durch einen Ansaugkäfig 24 Flüssigkeit ein und stößt diese durch die Öffnungen 25, 26 wieder aus. Die Pumpe 5 ist am distalen Ende eines Hohlkatheters 1 angeordnet, durch den die Antriebswelle 8 in Längsrichtung verläuft. An seinem Ende weist der Hohlkatheter 1 eine Einlaufschleuse 27 in Form eines Trichters auf, in die das Gehäuse 5 zum Entfernen der Pumpe aus einem Patientenkörper eingezogen werden kann. Eine Zugbewegung kann auf das Gehäuse 5 beispielsweise mittels der Züge 28, 29 aufgebracht werden, wobei die Züge 28 in Halterungen an der Außenseite des Hohlkatheters geführt sind, während der Zug 29 als im Inneren des Hohlkatheters verlaufend dargestellt ist.

Wenn die Führung der Züge 28, 29 eng genug ist, so können diese nicht nur eine Zugbewegung sondern auch einen Schub in Längsrichtung übertragen.

Die Züge 28, 29 können am proximalen Ende des Hohlkatheters 1, wie in der Fig. 8 unten dargestellt, beispielsweise in einem Klemmring 30 gehalten werden, der als ganzer zur Manipulation der Pumpe 5 entlang des Katheters verschoben oder auch gedreht und festgelegt werden kann. In dem Klemmring 30 sind die entsprechenden Züge 28 verklemmt.

In dem dargestellten Beispiel ist das Lager 20 ein zugfestes Drehlager, so dass das distale Ende des Rotors 6 in diesem Lager nicht nur drehbar gelagert sondern auch in Längsrichtung gehalten ist.

Das proximale Lager 23 erlaubt eine Bewegung der Welle 8 oder eines Wellenfortsatzes in Längsrichtung, so dass dort keine zugfeste Verbindung besteht.

Wird an der Antriebswelle 8 in Längsrichtung vom proximalen Ende des Hohlkatheters aus gezogen, so wird der Rotor 6 einer Längsdehnung unterworfen, die zu einer Querkompression führt.

Es ist auch denkbar, auf das Gehäuse 5 radialen Druck in Richtung der Pfeile 31, 32 auszuüben und damit eine Längsdehnung des Gehäuses zu erreichen, die sich auf den Rotor 6 übertragen lässt, indem das Gehäuse im Bereich des proximalen Lagers 23 an dem mit der Welle 8 fest verbundenen oder zumindest in Längsrichtung in Bezug auf die Welle 8 festgelegten Anschlag 33 anschlägt und bei einer weiteren Längsdehnung des Gehäuses den Rotor mit lang zieht.

Dadurch ergibt sich eine selbsttätige Querkompression des Rotors, so dass im Zuge des Zusammendrückens des Gehäuses 5 der Rotor einfach mitkomprimiert werden kann.

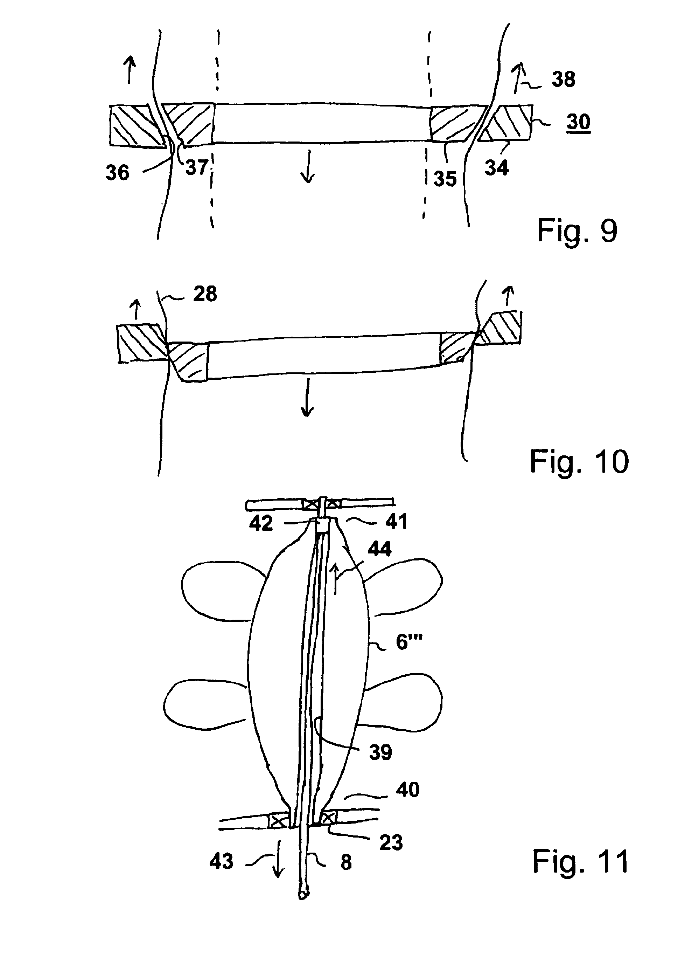

In Fig. 9 ist in schematischer Weise die Funktion des Klemmrings 30 gezeigt, der einen äußeren Teilring 34 und einen inneren Teilring 35 aufweist, die jeweils konische Begrenzungsflächen 36, 37 aufweisen. Wird der äußere Teilring 34 in Richtung des Pfeils 38 gegenüber dem inneren Teilring 35 bewegt, so ergibt sich das in Fig. 10 dargestellte Bild, bei dem die konischen Flächen 36, 37 aneinander zur Anlage kommen und sich verkeilen. Die zwischen ihnen angeordneten Züge 28 werden dabei verklemmt und in Längsrichtung festgelegt.

Der gesamte Klemmring 30 kann dann zur Manipulation der Züge 28 bewegt werden.

Mit Hilfe einer Vorrichtung, die nicht weiter ausgeführt ist, könnten auch der durchmesserreduzierte und der expandierte Zustand jeweils unabhängig von einander arretiert werden.

Die Fig. 11 zeigt schematisch eine besondere Ausgestaltung des Rotors 6''', der einen zentrischen Hohlraum 39 aufweist, durch den die Antriebswelle 8 längs von dem proximalen Ende 40 des Rotors zum distalen Ende 41 verläuft. Am distalen Ende 41 ist die Antriebswelle 8 mit dem Rotor über einen Fassungskörper 42 drehfest und zugfest verbunden, so dass der Rotor 6''' von dem proximalen Ende 40 aus über die Welle 8 antreibbar ist. Gleichzeitig kann aber der Rotor 6''' am proximalen Ende 40 mit der Welle 8 in der Zugrichtung, die durch den Pfeil 43 angedeutet ist, zugfest verbunden sein.

Dort ist der Rotor in dem proximalen Lager 23 drehbar und zugfest gelagert, so dass bei Aufbringen eines Drucks auf die Welle 8 in Richtung des Pfeils 44 der Rotor zwischen dem Fassungskörper 42 und dem Lager 23 in die Länge gezogen wird. Hierdurch kann der Rotor 6''' in Querrichtung komprimiert werden.

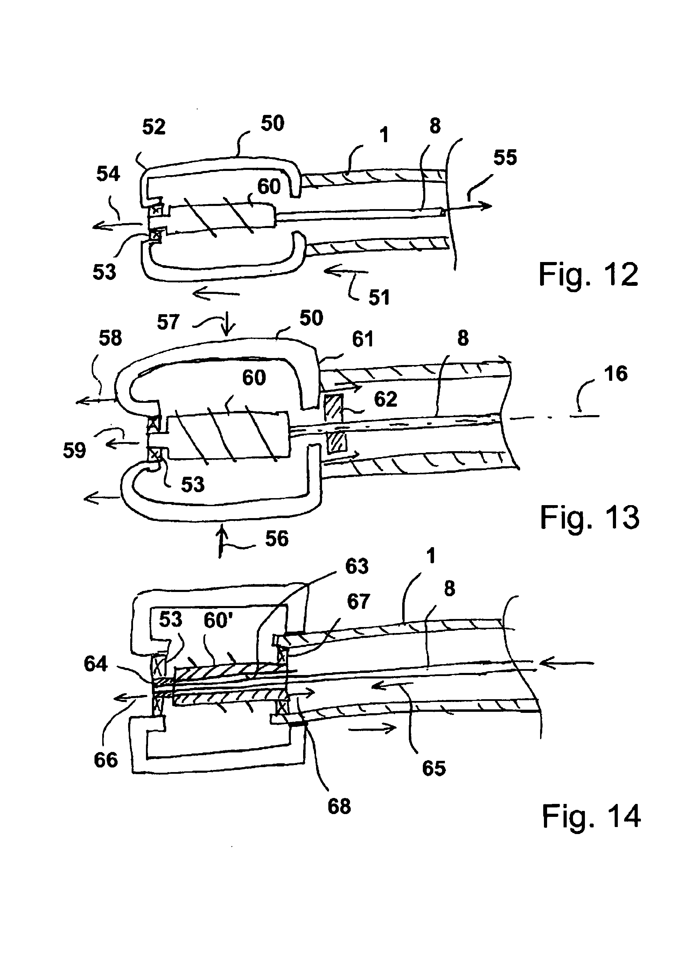

Die Fign. 12, 13 und 14 zeigen grundsätzlich in ähnlichen Ausgestaltungen unterschiedliche Prinzipien, nach denen ein Längszug auf einen Rotor aufgebracht werden kann. Dabei ist in der Fig. 12 das Gehäuse mit 50 bezeichnet. Dies umgibt den Rotor 60 und ist auf dem distalen Ende des Hohlkatheters 1 abgestützt.

Über den Hohlkatheter 1 kann somit ein Druck in Richtung des Pfeils 51 auf das Gehäuse 50 übertragen werden, dessen distales Ende 52 über das zugfeste Lager 53 einen Zug auf den Rotor 60 in Richtung des Pfeils 54 ausüben kann.

Am proximalen Ende des Rotors 60 ist die Antriebswelle 8 drehfest befestigt. Diese kann, was im Einzelnen nicht dargestellt ist, zudem axial verschieblich drehbar am Gehäuse 50 oder am Hohlkatheter 1 gelagert sein. Wird auf die Antriebswelle 8 in Richtung des Pfeils 55 ein Zug ausgeübt und gleichzeitig der Hohlkatheter gestützt, so wird der Rotor 60 in Längsrichtung gedehnt.

Anhand der Fig. 13 ist dargestellt, dass auf das Gehäuse 50 auch ein Querdruck senkrecht zur Längsrichtung, dargestellt durch die Pfeile 56, 57 ausgeübt werden kann, der wegen der bauchigen, in gewissem Maß steifen Ausgestaltung des Gehäuses zu einer Längsdehnung des Gehäuses 50 in Richtung der Längsachse 16 führt. Das Gehäuse kann aus einem Schlauch beispielsweise aus einem Elastomer oder aus Längsstäben bestehen, die mit einer Membran bespannt oder von einem verformbaren Schlauch umgeben sind. Das distale Ende des Gehäuses 50 dehnt sich in Richtung des Pfeils 58 aus. Auf den Rotor 60 wird in Richtung des Pfeils 59 über das zugfeste Drehlager 53 ein entsprechender Zug ausgeübt. Das Gehäuse 50 längt sich so weit in Richtung der Längsachse 16, bis sein proximales Ende 61 gegen den Anschlag 62 stößt, der unverschiebbar mit der Antriebswelle 8 verbunden ist. Bei entsprechender weiterer Ausdehnung des Gehäuses 50 in Längsrichtung wird ein Zug auf die Antriebswelle 8 mittels des Anschlags 62 ausgeübt, der zu einer Längsdehnung des Rotors 60 führt. Alternativ kann der Anschlag 62 auch weggelassen werden ,sofern ein Zug mittels der Antriebswelle 8 auf das proximale Ende des Rotors aufgebracht wird.

Die Fig. 14 zeigt, dass ein einen zentrischen Hohlraum 63 aufweisender Rotor 60' eingesetzt werden kann, wobei die Antriebswelle 8 den Hohlraum 63 von dessen proximaler Seite her bis zum distalen Ende durchsetzt. Dort ist die Antriebswelle 8 mit einem Verbindungselement 64 zugfest verbunden, das seinerseits drehbar und zugfest in dem Lager 53 gelagert sein kann. Das Lager kann als zugfestes oder nicht zugfestes Lager ausgebildet sein.

Mittels der Antriebswelle 8 kann in Richtung des Pfeils 65 auf den Rotor 60' ein Druck ausgeübt werden. Entsprechend wird über das Verbindungselement 64 ein Zug auf das distale Ende des Rotors 60' in Richtung des Pfeils 66 ausgeübt. Am proximalen Ende des Rotors 60' ist dieser zugfest in einem Lager 67 drehbar gelagert, das seinerseits am distalen Ende des Hohlkatheters 1 unverschiebbar befestigt ist. Entsprechend kann über dieses Lager 67 ein Zug auf das proximale Ende des Rotors in Richtung des Pfeils 68 aufgebracht werden, wodurch insgesamt der Rotor 60' in Längsrichtung gedehnt wird.
Alternativ kann der Rotor an seinem proximalen Ende auch zugfest im Gehäuse drehbar gelagert und das Gehäuse kann gegenüber dem Katheter axial festgelegt sein.

Durch die verschiedenen in Zusammenhang mit der Erfindung beschriebenen technischen Möglichkeiten, auf den Rotor eine Längsdehnung aufzubringen, wird dieser in Querrichtung komprimiert oder zumindest einfacher komprimierbar. Entsprechende Schaufelblätter können ebenfalls mitgedehnt und/oder in einen leichter komprimierbaren Zustand gebracht werden. Die Erfindung ermöglicht somit eine bessere Komprimierbarkeit des Rotors und insgesamt der Fluidpumpe.

## Patentansprüche

1. Fluidpumpe mit einem um seine Längsachse (16) drehbaren, antreibbaren Rotor (6,6', 6'', 6''', 60, 60'), der wenigstens ein Schaufelblatt (14, 14', 14'', 18) aufweist, **dadurch gekennzeichnet, dass** der Rotor wenigstens teilweise aus einem elastisch komprimierbaren und expandierbaren Material besteht und durch axial auf ihn wirkende Elemente in Richtung seiner Längsachse (16) elastisch dehnbar ist.

2. Fluidpumpe nach Anspruch 1, **dadurch gekennzeichnet, dass** ein Schub- und ein Zugelement (1,8) an verschiedenen Enden des Rotors (6, 6', 6'', 6''', 60, 60') und/oder eines Gehäuses (5,50) der Fluidpumpe angreifen.

3. Fluidpumpe nach Anspruch 1 oder 2, **dadurch gekennzeichnet, dass** der Rotor eine axial durchgehende, wenigstens teilweise materialelastische Nabe aufweist, mit der wenigstens ein Schaufelblatt wenigstens über einen Teil ihrer Länge verbunden ist.

4. Fluidpumpe nach Anspruch 1,2 oder 3, **dadurch gekennzeichnet, dass** der Rotor, insbesondere die Nabe, in Abhängigkeit von der Länge seinen Durchmesser ändert.

5. Fluidpumpe nach einem der Ansprüche 1, 2,3 oder 4, **dadurch gekennzeichnet, dass** die Durchmesser-änderung des Rotors durch eine vom Anwender hervorgerufenen Längenänderung des Rotors bzw. der Pumpe hervorgerufen wird.

6. Anordnung mit einer Fluidpumpe nach Anspruch 1 oder einem der folgenden, wobei der Rotor (6, 6', 6'', 6" ', 60, 60') an einem distalen Ende eines Katheters (1) angeordnet ist, **dadurch gekennzeichnet, dass** durch zwei entlang des Katheters verlaufende, gegeneinander bewegbare Elemente (1,8) auf beide Enden des Rotors (6, 6', 6", 6''', 60, 60') in Längsrichtung (16) ein Zug oder Druck in einander entgegengesetzten Richtungen aufbringbar ist.

7. Anordnung nach Anspruch 6, **dadurch gekennzeichnet, dass** eines der Elemente eine Hülle und das andere Element eine in der Hülle verlaufende Seele ist.

8. Anordnung nach Anspruch 7, **dadurch gekennzeichnet, dass** die Hülle durch den Katheter (1) gebildet ist.

9. Anordnung nach Anspruch 7 oder 8, **dadurch gekennzeichnet, dass** die Seele durch eine Antriebswelle (8) gebildet ist.

10. Anordnung nach Anspruch 7, 8 oder 9, **dadurch gekennzeichnet, dass** das proximale Ende des Rotors (6, 6', 6'', 60) mit der Seele (8) und das distale Ende des Rotors mit einem Gehäuse (5,50) des Rotors zugfest verbunden sind und dass das Gehäuse mit der Hülle (1) druckfest verbunden ist.

11. Anordnung nach Anspruch 7, 8 oder 9, **dadurch gekennzeichnet, dass** das proximale Ende des Rotors (6''', 60') mit dem Ende der Hülle (1) unmittelbar oder mittelbar über das Gehäuse (5,50) zugfest verbunden ist und dass das distale Ende des Rotors mit der Seele (8) unmittelbar oder mittelbar über das Gehäuse (5,50) druckfest verbunden ist.

12. Anordnung mit einer Fluidpumpe nach Anspruch 1 oder einem der folgenden, **dadurch gekennzeichnet, dass** der Rotor von einem ebenfalls in Richtung der Längsachse dehnbaren Gehäuse (5,50) umgeben ist.

13. Anordnung nach Anspruch 12,**dadurch gekennzeichnet, dass** das Gehäuse in dem Zustand, in dem keine Kräfte auf es in Längsrichtung wirken, eine bauchige Form mit einem für den expandierten Rotor ausreichenden Innenraum aufweist.

14. Anordnung nach Anspruch 12 oder 13, **dadurch gekennzeichnet, dass** das Gehäuse (5,50) im Falle einer Querkompression im Wesentlichen senkrecht zur Längsachse (16) eine Längsdehnung parallel zur Längsachse erfährt.

15. Anordnung mit einer Fluidpumpe nach Anspruch 1 oder einem der folgenden, **dadurch gekennzeichnet, dass** eine Schleuse (2,27) vorgesehen ist, in die die Fluidpumpe zurückziehbar ist mit einer Vorrichtung zur Radialkompression des Gehäuses (5,50).

16. Anordnung nach Anspruch 15, **dadurch gekennzeichnet, dass** die Vorrichtung zur Radialkompression als Einlauftrichter (27) ausgeführt ist.

17. Fluidpumpe nach Anspruch 1 oder Anordnung nach Anspruch 6 oder einem der folgenden, **dadurch gekennzeichnet, dass** das wenigstens eine Schaufelblatt (14, 14', 14'', 18), des Rotors (6, 6', 6'', 6''', 60, 60') durch die Längsdehnung des Rotors ebenfalls in Längsrichtung des Rotors gedehnt wird und insbesondere seine Formstabilität wenigstens teilweise verliert.
